# EUROPÄISCHE PATENTSCHRIFT

(11) **EP 0 597 060 B1**
(45) Veröffentlichungstag und Bekanntmachung des Hinweises auf die Patenterteilung: **16.04.1997**
(21) Anmeldenummer: 93909906.5
(22) Anmeldetag: 06.05.1993
(51) Int. Cl.: G01F 1/66, A61B 5/087

(54) **SPIROMETER, INSBESONDERE ULTRASCHALL-SPIROMETER**
SPIROMETER, IN PARTICULAR ULTRASOUND SPIROMETER
SPIROMETRE, NOTAMMENT SPIROMETRE A ULTRA-SONS

(30) Priorität: 03.06.1992 DE 4218317; 07.07.1992 DE 4222286
(43) Veröffentlichungstag der Anmeldung: 18.05.1994
(73) Patentinhaber: NDD Medizintechnik GmbH, D-97003 Würzburg (DE)
(72) Erfinder: HARNONCOURT, Karl, A-8010 Graz (AT)
(74) Vertreter: Laufhütte, Dieter, Dr.-Ing.
(86) Internationale Anmeldenummer: EP9301109
(87) Internationale Veröffentlichungsnummer: WO9324810

(56) Entgegenhaltungen:
- PATENT ABSTRACTS OF JAPAN vol. 10, no. 30 (P-426) 5. Februar 1986 & JP-A-60 181 616
- PATENT ABSTRACTS OF JAPAN vol. 7, no. 290 (P-245) 24. Dezember 1983 & JP-A-58 162 815
- IEEE TRANSACTIONS ON BIO-MEDICAL ENGINEERING Bd. 33, Nr. 8, August 1986, New York, US, SS 768-773; C. BUESS: 'Design and Construction of a Pulsed Ultrasonic Air Flowmeter'
- PATENT ABSTRACTS OF JAPAN vol. 10, no. 154 (P-463) 4. Juni 1986 & JP-A-61 007 416
- PATENT ABSTRACTS OF JAPAN vol. 10, no. 177 (P-470) 21. Juni 1986 & JP-A-61 025 021

## Beschreibung

Die Erfindung betrifft ein Spirometer, insbesondere ein Ultraschall-Spirometer, bei dem ein Sender-/Empfangszellenpaar in einer Meßstrecke schräg oder senkrecht zur Meßrohrachse angeordnet ist.

Die Spirometrie, d. h. die Meßung der Luftbewegungen bei der Atmung, erfolgt in der Regel über Meßgeräte, die den Volumenstrom eines Gases in einem Rohr durch die Bestimmung der Strömungsgeschwindigkeit messen. Der Volumenstrom ergibt sich dann aus dem Produkt aus Querschnittsfläche des Rohres und der mittleren Strömungsgeschwindigkeit. Bei der Anwendung eines solchen Meßgerätes zur Untersuchung der Leistungsfähigkeit der menschlichen Lunge ist der zeitliche Verlauf des Volumenstroms beim Einatmen und beim Ausatmen von Interesse. Durch Integration kann die in einem bestimmten Zeitintervall eingeatmete oder ausgeatmete Luftmenge bestimmt werden. Es werden zur Zeit verschiedene Methoden der Strömungsmessung in der Lungenfunktionsdiagnostik (Pneumotachografie) verwendet. Die Messung von Staudrucken vor konstanten Widerständen (zum Beispiel Metallnetze) oder in Pneumotachographen nach Fleisch, die Messung mit Propeller, die Messung mit Thermistor und andere Verfahren sind bekannt.

Aus der JP 60-117149 A und aus der CH 669463 A5 sind sogenannte Ultraschall-Spirometer der eingangs genannten Gattung bekannt, bei denen ein Sender-/Empfangszellenpaar in einer Meßstrecke schräg zur Meßrohrachse angeordnet ist. Bei diesen bekannten Ultraschall-Spirometern wird die Strömungsgeschwindigkeit über Ultraschall-Dopplermeßung ermittelt. Diese Meßtechnik erlaubt eine präzise Vermessung der Strömungsfelder innerhalb der Meßrohrachse und damit eine genaue Ermittlung des Volumenstroms. Bei der Spirometrie haben diese Meßgeräte aber den Nachteil, daß das Meßrohr bei jeder Messung mit Keimen und dergleichen kontaminiert wird. Daher ist es erforderlich, das Meßrohr nach jeder Messung zu desinfizieren, was einerseits aufwendig ist, andererseits aber bei unsachgemäßer Durchführung eine ständige Quelle der Infektionsgefahr darstellt.

Eine Lösung dieses Hygieneproblems wurde durch Entwicklung von sogenannten Wegwerfsensoren versucht. Demgemäß wurde bereits versucht, das von dem Atemgas durchströmte Meßrohr mit einer düsenförmigen Verengung zu versehen, wobei etwa an der engsten Stelle der Verengung ein Kanal in das Rohr einmündet, der einen Anschluß für die Meßeinrichtung aufweist. Dieses Rohr mit der düsenförmigen Verengung, das mit der Atemluft kontaminiert wird, soll dann als Wegwerfteil ausgestaltet sein, das nach jeweils einmaligem Gebrauch ausgetauscht werden kann. Diese unter hygienischen Gesichtspunkten gute Lösung bedingt aber auf Grund der Verengung im Rohr eine Störung der zu vermessenden Strömung und somit ein verfälschtes Meßergebnis.

JP-A-6 0181616 beschreibt eim Flowmeter gemäß Oberbegriff des Anspruchs 1.

JP-A-6100 M7416 beschreibt eim aufklappbarer Meßgerät dieser Art.

Aufgabe der vorliegenden Erfindung ist es, einen gattungsgemäßen Spirometer derart weiterzubilden, daß eine störungsfreie Strömungsgeschwindigkeitsmessung unter Sicherstellung der notwendigen Hygiene ermöglicht ist.

Erfindungsgemäß wird diese Aufgabe durch die Merkmale des Anspruchs 1 gelöst. Demnach wird in das Meßrohr des Ultraschall-Spirometers paßgenau ein gegebenenfalls steriles, leicht austauschbares Rohr eingesetzt. Am Übergang zur Meßstrecke weist dieses Meßfenster in der Art auf, daß in den entsprechenden Öffnungen Einsätze eingesetzt sind, die einerseits durchlässig für Schallwellen, aber weitgehend undurchlässig für Keime sind. Dieser Lösung liegt die Erkenntnis zugrunde, daß in dem Bereich, in welchem die Meßstrecke in die Meßrohrachse einmündet, ultra-schalldurchlässige Fenster vorhanden sein müssen, da die Wandung eines eingesetzten Rohres die Ultraschallwellen zu stark dämpfen würde und damit eine Ultraschall-Dopplermeßung unmöglich machen würde. Andererseits wird durch die Einsätze innerhalb der Meßfenster sichergestellt, daß möglichst keine Keime und auch möglichst keine sonstige Verschmutzung in die Meßstrecke eindringen können, was wiederum eine aufwendige Reinigung der Meßapparatur nach sich ziehen würde.

Gemäß einer besonders vorteilhaften Ausführungsform der Erfindung bestehen die Einsätze aus elastischem Kunststoff, insbesondere Schaumgummi. Experimentelle Untersuchungen haben ergeben, daß die Elastizität von Schaumgummi eine Übertragung der Ultraschallwellen durch das Meßfenster hindurch zuläßt. Andererseits sind die durch den Schaumgummieinsatz verschlossenen Meßfenster für die in der Atemluft enthaltenen Keime weitgehend undurchlässig. Die Poren des Schaumgummis weisen eine labyrinthartige, offene Kommunikation auf und bilden daher keinen Verschluß, wie beispielsweise eine dichte Membran. Die Poren werden im Gebrauch nicht durchströmt, da der Sensor, der hinter dem Schaumgummifenster angeordnet ist, in einem Blindsack angeordnet ist. Dabei können die Einsätze aus elastischem Kunststoff und insbesondere aus Schaumgummi zusätzlich mit einem keimtötenden Mittel getränkt sein.

Um die Meßfenster lagegenau in den Spirometer einsetzen zu können, ist dieser in einer durch das Meßrohr verlaufenden Ebene derart geteilt sein, daß er in dieser durch das Meßrohr gehenden Ebene aufklappbar ist. In den entsprechend aufgeklappten Spirometer läßt sich das sterile Rohr nach Entfernung der Sterilverpackung lagegenau einsetzen. Dazu können die Zentrierungen als Zentriernasen bzw. Zentrierflansche an dem Meßrohr vorgesehen sein, die zusätzlich beim Zuklappen des Ultraschall-Spirometers ein Verrutschen des Rohres verhindern.

Eine weitere Lösung der Erfindung besteht darin, daß die Einsätze aus sehr dünnen Platten bestehen. Diese müssen im Vergleich zu den erzeugten Wellenlängen so dünn sein, daß sie durch die Ultraschallwellen in Schwingungen versetzbar sind. Hierzu eignen sich beispielsweise sogenannte Mylarfolien. In diesem Fall sind die Platten für die Ultraschallwellen durchlässig. Hier kann das sterile Rohr zwei gegenüberliegende, parallele Flächen aufweisen, deren Breite idealerweise der Breite der in ihrer Form rechteckigen Platten entspricht. Die die Einsätze bildenden, rechteckigen Platten sind co-planar mit den entsprechenden parallelen Flächen.

Damit die beiden parallelen Flächen lagegenau zum Meßfenster ausgerichtet sind, kann das sterile Rohr wiederum Zentrierungen zum lagegenauen Einsetzen aufweisen. Diese können beispielsweise aus einem Flansch mit einer Zentrierausnehmung bestehen. Weitere Einzelheiten und Vorteile der vorliegenden Erfindung werden anhand von in der Zeichnung dargestellten Ausführungsformen im folgenden näher erläutert. Es zeigen:
- Fig. 1:: eine Schnittdarstellung eines Ultraschall-Spirometers gemäß einer ersten Ausführungsform der vorliegenden Erfindung und
- Fig. 2:: einen Teil des erfindungsgemäßen Ultraschall-Spirometers gemäß einer zweiten Ausführungsform der vorliegenden Erfindung in drei Ansichten.

Der Aufbau des Ultraschall-Spirometers 10 entspricht im wesentlichen demjenigen, der in der CH 669463 A5 bereits beschrieben wurde. Die zentralen Teile dieses Ultraschall-Spirometers bestehen aus dem Meßrohr 12 für die Atemluft und der schräg dazu angeordneten Meßstrecke 16. In entsprechenden Kammern 30 und 34 sind die Sende- und Empfangselemente 32 und 36 angeordnet.

In das Meßrohr 12 ist paßgenau ein steriles oder gegebenenfalls nahezu steriles Rohr 14 eingesetzt, das am Übergang zur Meßstrecke 16 Meßfenster in der Art aufweist, daß in entsprechenden Öffnungen Einsätze 18 aus Schaumgummi eingesetzt sind. Diese Schaumgummieinsätze 18 sind randseitig auf die entsprechenden Öffnungen des Meßrohres 12 aufgeklebt und ragen in den jeweiligen schräg abgehenden Kanal der Meßstrecke 16 hinein.

Das weitgehend sterile Rohr 14 kann aus einem beliebigen Material bestehen. So ist beispielsweise an den Einsatz von Kunststoffröhrchen gedacht. Vorteilhaft wäre der Einsatz von einem einfach verrottbaren Kunststoff. Grundsätzlich ist auch der Einsatz von Papierröhrchen oder von Röhrchen aus anderen sterilisierbaren Werkstoffen, die verrottbar sind, möglich.

Die Schaumgummieinsätze können mit einem keimtötenden Mittel getränkt sein.

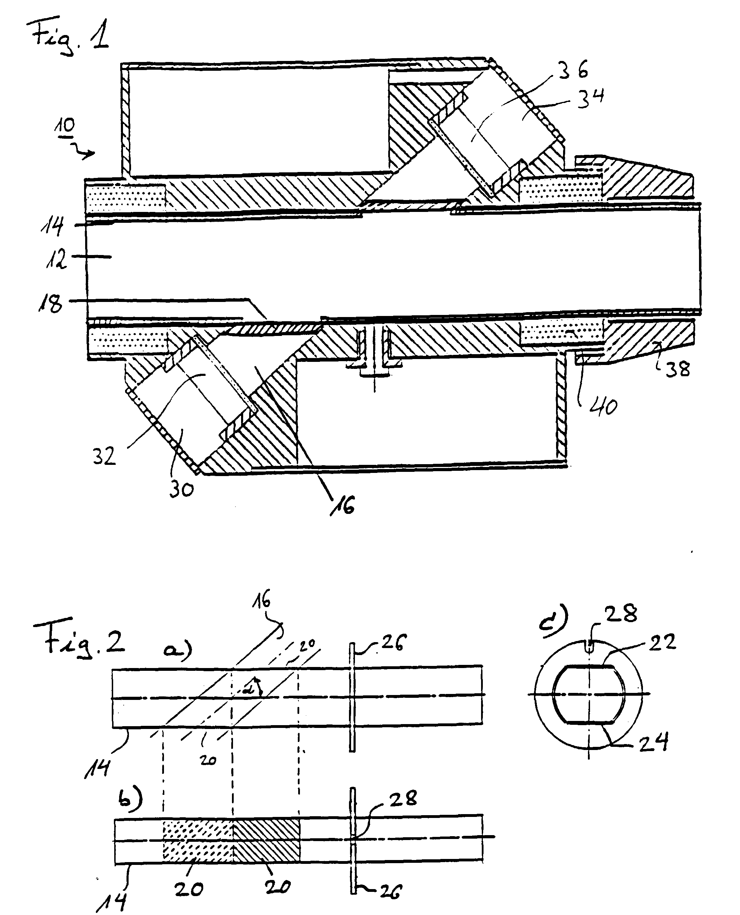

Der Ultraschall-Spirometer gemäß Fig. 1 ist in nicht näher dargestellter Art und Weise in der Ebene des Meßrohres 12 geteilt. Hierdurch kann er zum Einlegen und zum Entnehmen des sterilen Rohres 14 aufgeklappt werden.

Bei der in Fig. 2 dargestellten Ausführungsform ist das sterile Rohr 14 im Unterschied zur ersten Ausführungsform nicht mit einem kreisrunden Querschnitt gebildet. Wie aus der Ansicht gemäß Fig. 2c ersichtlich ist, weist das sterile Rohr 14 jeweils zwei planparallele Seitenflächen 22 und 24 auf. Aus Fig. 2b ist zu ersehen, daß innerhalb dieser planparallelen Platten 22 und 24 jeweils rechteckige Platten 20 zur Abdeckung der Meßfenster eingesetzt sind. Diese Platten 20 verlaufen co-planar zu den parallelen Ebenen 22 und 24. Entscheidend für die Funktionsfähigkeit des Ultraschall-Spirometers ist es, daß die Platten 20 im Vergleich mit den erzeugten Ultraschallwellen so dünn sind, daß sie durch die Ultraschallwellen in Schwingungen versetzbar sind, so daß sich die Ultraschallwellen auch über das Fenster in dem sterilen Rohr 14 hinweg fortsetzen können. In dem hier dargestellten Ausführungsbeispiel entspricht die Breite der rechteckigen Platten 20 auch der Breite der parallelen Flächen 22 und 24 des sterilen Rohres 14.

In Fig. 2a ist die Lage der Meßfenster 20 nochmals angegeben, wobei zur Verdeutlichung der Verlauf der um einen Winkel alpha geneigten Meßstrecke 16 eingezeichnet ist. Das sterile Meßrohr weist einen Flansch 26 mit einer Zentrierausnehmung 28 auf. Wird das Meßrohr 14 gemäß der Ausführungsform nach Fig. 2 in einem Ultraschall-Spirometer nach Fig. 1 eingesetzt, so kann dies auf Grund der Ausführung des Flansches 26 ohne Aufklappen des Spirometers einfach dadurch erfolgen, daß der Flansch 38 und das ringförmige Teil 40 abgeschraubt bzw. herausgenommen werden und das sterile Rohr 14 eingesetzt wird. Die genaue Lage des sterilen Rohres 14 im Meßrohr 12 wird durch Eingriff eines vorrichtungsseitigen Vorsprungs in die Zentrierausnehmung 28 am Flansch 26 des sterilen Rohrs 14 sichergestellt.

Mit der vorliegenden Erfindung wird ein genauer, spirometrischer Sensor an die Hand gegeben, welcher den Atemstrom nicht behindert und ein im übrigen - vorteilhaft verrottbares - von der Masse her möglichst kleines Wegwerfteil beinhaltet, das eine sichere hygienische Barriere ermöglicht.

## Patentansprüche

1. Spirometer, insbesondere Ultraschall-Spirometer, bei dem ein Sender/Empfangszellenpaar in einer Meßstrecke schräg oder senkrecht zur Meßrohrachse angeordnet ist, wobei in das Meßrohr (12) paßgenau ein leicht austauschbares Rohr (14) eingesetzt ist, das am Übergang zur Meßstrecke (16) Meßfenster in der Art aufweist, daß in entsprechenden Öffnungen des Meßrohres (12) Einsätze (18, 20) eingesetzt sind, die durchlässig für Schallwellen, aber weitgehend undurchlässig für Keime und Verschmutzungen sind, dadurch gekennzeichnet daß das Meßrohr (12) geteilt ist, so daß durch Aufklappen des Spirometers (10) das weitgehend sterile Rohr (14) lagegenau einsetzbar ist und daß das weitgehend sterile Rohr (14) Zentrierungen (28) zum lagegenauen Einsetzen in das Meßrohr (12) aufweist.

2. Spirometer nach Anspruch 1, dadurch gekennzeichnet, daß die Einsätze (18) aus elastischem Kunststoff bestehen.

3. Spirometer nach Anspruch 2, dadurch gekennzeichnet, daß die Einsätze (18) aus Schaumgummi bestehen.

4. Spirometer nach einem der Ansprüche 1-3, dadurch gekennzeichnet, daß die Einsätze (10, 18) mit einem keimtötenden Mittel getränkt sind.

5. Spirometer nach Anspruch 1, dadurch gekennzeichnet, daß die Einsätze aus Platten (20) bestehen, die im Vergleich mit den erzeugten Wellenlängen so dünn sind, daß sie durch die Ultraschall-Wellen in Schwingung versetzbar sind.

6. Spirometer nach Anspruch 5, dadurch gekennzeichnet, daß die Platten (20) aus Polyethylentherephthalat-Folien bestehen.

7. Spirometer nach Anspruch 5 oder 6, dadurch gekennzeichnet, daß das weitgehend sterile Rohr (14) zwei gegenüberliegende parallele Flächen (20, 22) aufweist, deren Breite der Breite der rechteckigen Platten (20) entspricht.

8. Spirometer nach einem der Ansprüche 1-7, dadurch gekennzeichnet, daß das weitgehend sterile Rohr (14) einen flansch (26) mit einer Zentrierausnehmung (28) aufweist.

## Claims

1. Spirometer, in particular ultrasound spirometer, in which a transmitter/receiver cell pair is arranged in a measuring path, obliquely or perpendicularly to the axis of the measuring tube, an easily interchangeable tube (14) being fitted accurately into the measuring tube (12) and having, at the junction with the measuring path (16), measuring windows such that inserts (18, 20) are fitted into corresponding openings of the measuring tube (12), which inserts are transparent to soundwaves but substantially impermeable to germs and contamination, characterized in that the measuring tube (12) is split, so that by tilting open the spirometer (10), the substantially sterile tube (14) can be fitted in the correct position, and in that the substantially sterile tube (14) has centrers (28) for fitting into the measuring tube (12) in the correct position.

2. Spirometer according to Claim 1, characterized in that the inserts (18) consist of elastic polymer.

3. Spirometer according to Claim 2, characterized in that the inserts (18) consist of foam rubber.

4. Spirometer according to one of Claims 1-3, characterized in that the inserts (10, 18) are impregnated with a germicidal agent.

5. Spirometer according to Claim 1, characterized in that the inserts consist of plates (20) which are so thin, in comparison with the wavelengths generated, that they can be displaced in oscillation by the ultrasound waves.

6. Spirometer according to Claim 5, characterized in that the plates (20) consist of polyethylene terephthalate sheets.

7. Spirometer according to Claim 5 or 6, characterized in that the substantially sterile tube (14) has two opposite parallel faces (20, 22) whose widths correspond to the widths of the rectangular plates (20).

8. Spirometer according to one of Claims 1-7, characterized in that the substantially sterile tube (14) has a flange (26) with a centring aperture (28).

## Revendications

1. Spiromètre, notamment spiromètre à ultra-sons, dans lequel une paire de cellules d'émission/réception est disposée dans un trajet de mesure qui est incliné ou perpendiculaire à l'axe du tube de mesure, et dans le tube de mesure (12) est placé avec un ajustage précis un tube facilement échangeable (14) qui présente à la transition vers le trajet de mesure (16) des fenêtres de mesure de manière que dans les ouvertures correspondantes du tube de mesure (12) soient placés des inserts (18, 20) qui laissent passer les ondes sonores mais qui sont sensiblement imperméables aux germes et aux saletés, caractérisé en ce que le tube de mesure (12) est divisé de telle sorte qu'en dépliant le spiromètre (10), le tube sensiblement stérile (14) peut être mis en place avec une précision de position et en ce que le tube sensiblement stérile (14) présente des centrages (28) pour la mise en place avec une précision de position dans le tube de mesure (12).

2. Spiromètre selon la revendication 1, caractérisé en ce que les inserts (18) sont réalisés en une matière synthétique élastique.

3. Spiromètre selon la revendication 2, caractérisé en ce que les inserts (18) sont en caoutchouc mousse.

4. Spiromètre selon l'une des revendications 1 à 3, caractérisé en ce que les inserts (10, 18) sont imprégnés d'un produit germicide.

5. Spiromètre selon la revendication 1,caractérisé en ce que les inserts sont constitués de plaques (20) qui, comparées aux longueurs d'ondes produites, sont suffisamment minces pour qu'elles puissent être amenées à osciller par les ondes ultra-sonores.

6. Spiromètre selon la revendication 5, caractérisé en ce que les plaques (2) sont constitués de feuilles en polyéthylènetéréphtalate.

7. Spiromètre selon la revendication 5 ou 6, caractérisé en ce que le tube sensiblement stérile (14) présente deux surfaces parallèles opposées (20, 22) dont la largeur correspond à la largeur des plaques rectangulaires (20).

8. Spiromètre selon l'une des revendications 1-7, caractérisé en ce que le tube sensiblement stérile (14) présente un collet (26) avec un évidement de centrage (28).
